(19) Europäisches Patentamt
European Patent Office
Office européen des brevets

(11) Numéro de publication: **0 369 881 A1**

(12) **DEMANDE DE BREVET EUROPEEN**

(21) Numéro de dépôt: **89403142.6**

(51) Int. Cl.[5]: **C07J 31/00, A61K 31/565**

(22) Date de dépôt: **15.11.89**

(30) Priorité: **16.11.88 FR 8814868**

(43) Date de publication de la demande: **23.05.90 Bulletin 90/21**

(84) Etats contractants désignés: **CH DE GB IT LI NL**

(71) Demandeur: **ROUSSEL-UCLAF**
**35, boulevard des Invalides**
**F-75007 Paris(FR)**

(72) Inventeur: **Hardy, Michel**
**3, Domaine du Château Gaillard**
**F-94700 Maisons Alfort(FR)**
Inventeur: **Nique, François**
**7, Allée Pierre Brossolette**
**F-93320 Pavillon sous Bois(FR)**
Inventeur: **Philibert, Daniel**
**16, Rue Chevalier**
**F-94210 La Varenne Saint Hilaire(FR)**

(74) Mandataire: **Bourgouin, André et al**
**Département des Brevets ROUSSEL UCLAF**
**111, route de Noisy B.P. no 9**
**F-93230 Romainville(FR)**

(54) **Nouveaux produits dérivés de la structure 3-céto delta-4,9 19-nor stéroides et leur application comme médicaments.**

(57) L'invention a pour objet les produits de formule ($I_{AD}$) :

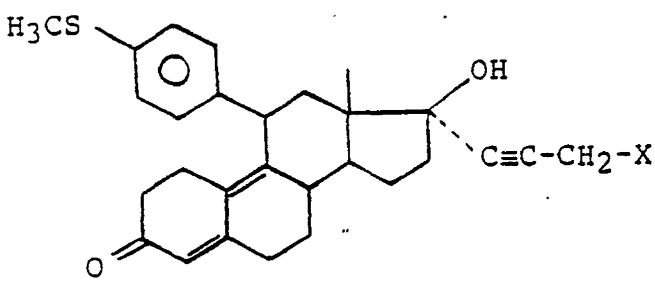

($I_{AD}$)

dans laquelle X = OH ou halogène.
Leur application comme médicaments et les compositions les renfermant.

EP 0 369 881 A1

# Nouveaux produits dérivés de la structure 3-céto delta-4,9 19-nor stéroïdes et leur application comme médicaments.

Dans sa demande de brevet japonais déposée le 1er mars 1983 sous le n° 3 190 983, la demanderesse a décrit de nouveaux produits dérivés de la structure 3-céto delta-4,9 19-nor stéroïdes de formule (I) :

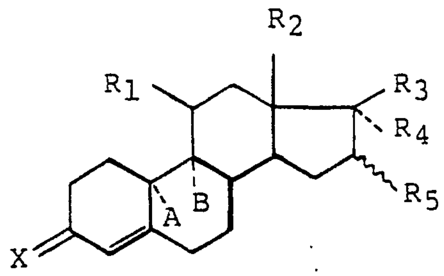

(I)

dans laquelle soit $R_1$ représente un radical thiényle éventuellement substitué, un radical furyle ; un radical cycloalkyle ayant de 3 à 6 atomes de carbone ; un radical phényle éventuellement substitué par un ou plusieurs radicaux choisis parmi les radicaux hydroxy, halogène, trifluorométhyle, alkyle, alkyloxy, alkylthio éventuellement oxydé sous forme de sulfoxyde ou de sulfone, ou alkényloxy ayant au plus 6 atomes de carbone et phénoxy soit :

$R_1$ représente un radical naphtyle ou diphényle ou un radical alkyle ou alkényle portant éventuellement plusieurs insaturations ayant au plus 6 atomes de carbone ;

$R_2$ représente un radical méthyle ou éthyle ;

$R_3$ représente un atome d'hydrogène, un radical alkyle, alkényle ou alkynyle éventuellement substitué, un radical hydroxy, acétyle, hydroxyacétyle, carboxyalkyloxy ayant de 2 à 4 atomes de carbone éventuellement estérifié ou salifié, hydroxyalkyle éventuellement estérifié ;

$R_4$ représente un atome d'hydrogène, un radical hydroxy, un radical alkyle, alkényle ou alkynyle ayant au plus 12 atomes de carbone éventuellement substitué par un radical alkylamino, dialkylamino, ou par un halogène, un radical alkylthio, alkyloxy ou trialkylsilyle ;

$R_5$ représente un atome d'hydrogène ou un radical méthyle en position alpha ou béta ;

X représente un atome d'oxygène ou un radical hydroxyimino ou alkyloxyimino ayant de 1 à 4 atomes de carbone en position syn ou anti ;

A et B représentent une fonction alpha époxy ou la présence d'une seconde liaison entre les carbones 9 et 10, ainsi que les sels de ces produits avec les acides lorsque $R_4$ représente un radical comportant une fonction amino.

A l'exception de ces produits dans lesquels A et B représentent une seconde liaison entre les carbones qui les portent, X représente un atome d'oxygène,

$R_5$ représente un atome d'hydrogène et :

a) $R_2$ représente un radical méthyle et :

a-1) $R_3$ représente un radical hydroxy et :

a-1-1) $R_1$ représente un radical éthyle ou phényle et $R_4$ représente un atome d'hydrogène, ou

a-1-2) $R_1$ représente un radical éthyle, propyle, isopropyle, vinyle, allyle, isopropényle, phényle, 4-fluoro phényle, 2 ou 3-méthoxy phényle ou thiényle et $R_4$ représente un radical éthynyle, ou :

a-1-3) $R_1$ représente un radical propyle, isopropyle, vinyle, allyle, isopropényle, 4-méthoxy phényle ou thiényle et $R_4$ représente un radical méthyle,

a-2) $R_3$ représente un radical acétyle et :

a-2-1) $R_1$ représente un radical éthyle, vinyle ou phényle et $R_4$ représente un radical hydroxy ou :

a-2-2) $R_1$ représente un radical vinyle et $R_4$ représente un radical méthyle.

b) $R_2$ représente un radical éthyle et :

$R_1$ représente un radical vinyle, $R_3$ représente un radical hydroxy et $R_4$ représente un atome d'hydrogène.

La demanderesse a également décrit un procédé de préparation de ces produits ainsi que leur application comme médicaments et les compositions pharmaceutiques renfermant ces médicaments.

Parmi les produits de formule (I), la demanderesse a décrit, à l'exemple 23 de la demande citée

précédemment, la l7béta-hydroxy 11béta-/4-(méthylthio) phényl/ 17-alpha-(1-propynyl) estra-4,9-dièn-3-one de formule :

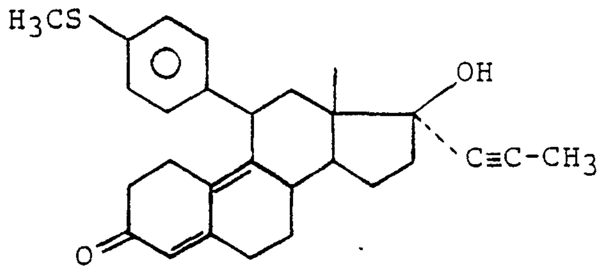

La présente demande a pour objet des nouveaux produits répondant à la formule générale de la demande citée précédemment et apparentés au produit de l'exemple 23, mais non décrits dans ladite demande.

La présente demande concerne les produits de formule ($I_{AD}$):

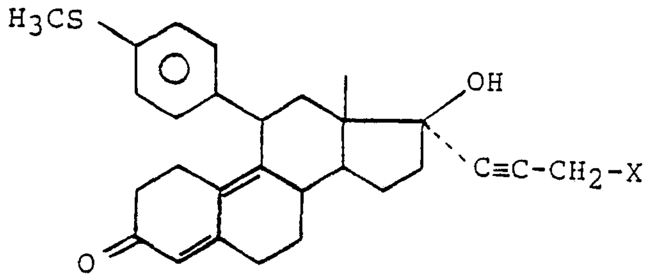

($I_{AD}$)

dans laquelle le substituant X représente un radical hydroxy ou un atome d'halogène.

Par atome d'halogène on entend bien entendu le fluor, le chlore, le brome et l'iode.

Parmi les produits de formule ($I_{AD}$) on préfère ceux dans lesquels X représente un atome de fluor, de chlore ou de brome et spécialement celui dans lequel X représente un atome de fluor.

Les produits de formule ($I_{AD}$) peuvent être préparés par les méthodes indiquées dans la demande japonaise n° 3 190 983. Des exemples de réalisation des produits de l'invention sont donnés ci-après dans la partie expérimentale. Le produit de formule ($I_{AD}$) dans lequel X représente un atome d'iode peut être préparé par exemple en faisant agir, sur le produit de l'exemple 2 un iodure alcalin tel que l'iodure de sodium au sein d'un solvant comme l'acétone.

Les produits de formule ($I_{AD}$) de la présente demande sont des produits particulièrement intéressants du point de vue pharmacologique ; ils possèdent en particulier une remarquable activité antiglucocorticoïde comme le montrent les résultats des tests exposés ci-après.

L'étude des produits sur les récepteurs hormonaux a permis de mettre en évidence des activités progestomimétiques ou anti-progestomimétiques, androgènes ou anti-androgènes.

Les produits de formule ($I_{AD}$) peuvent donc être utilisés comme médicaments pour lutter principalement contre les effets secondaires des glucocorticoïdes, ils permettent de lutter également contre les troubles dus à une hypersécrétion de glucocorticoïdes et notamment contre le vieillissement en général et plus particulièrement contre l'hypertension, l'athérosclérose, l'ostéoporose, le diabète, l'obésité ainsi que l'immunodépression et l'insomnie.

Les produits de formule ($I_{AD}$) qui possèdent des propriétés anti-progestomimétiques peuvent être utilisés comme contraceptifs originaux ; ils peuvent être utilisés contre les dérèglements hormonaux ; ils peuvent par ailleurs présenter un intérêt dans le traitement des cancers hormono-dépendants.

Certains produits de formule ($I_{AD}$) peuvent également présenter des propriétés progestomimétiques et peuvent ainsi être employées dans le traitement des aménorrhées, des dysménorrhées, des insuffisances lutéales et de l'endométriose.

Les produits de formule ($I_{AD}$) qui présentent des propriétés anti-androgènes peuvent être utilisés dans le traitement des hypertrophies et du cancer de la prostate, de l'hyperandrogènie, de l'anémie, de l'hirsutisme et de l'acné.

L'invention a donc pour objet à titre de médicament les produits de formule ($I_{AD}$) pharmaceutiquement

acceptables, c'est-à-dire non toxiques aux doses utilisées.

L'invention a plus spécialement pour objet, à titre de médicament la 17alpha-(3-fluoro 1-propynyl) 17béta-hydroxy 11béta-[4-(méthylthio) phényl] estra-4,9-dien-3-one.

La posologie utile varie en fonction de l'affection à traiter et de la voie d'administration ; elle peut varier par exemple de 10 mg à 1 g par jour chez l'adulte par voie orale.

Les nouveaux produits de formule ($I_{AD}$), tels que définis ci-dessus peuvent être employés pour préparer des compositions pharmaceutiques renfermant, à titre de principe actif, l'un au moins desdits produits.

Les produits de formule ($I_{AD}$) sont utilisés par voie digestive, parentérale ou locale. Ils peuvent être prescrits sous forme de comprimés simples ou dragéifiés, de gélules, de granulés, de suppositoires, de préparations injectables, de pommades, de crèmes, de gels, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétales, les dérivés paraffiniques, les glycols, les divers agents mouillants, dispersants ou émulsifiants, les conservateurs.

L'invention a donc pour objet les compositions pharmaceutiques renfermant comme principe actif au moins un produit de formule ($I_{AD}$).

Les exemples suivants illustrent l'invention :

**EXEMPLE 1 : 17béta-hydroxy 17alpha-(3-hydroxy 1-propynyl) 11béta-[4-(méthylthio) phényl] estra-4,9-dien-3-one**

A un mélange agité et refroidi à 0°/+5°C de 4,7 g de (1,2-éthanediyl) acétal cyclique de 5alpha, 10alpha-époxy 17béta-hydroxy 17alpha-[3-(2-tétrahydropyrannyloxy) 1-propynyl] estr-9(11)-èn-3-one décrit dans la demande de brevet international publiée sous le n° WO 87/05908, 15 $cm^3$ de tétrahydrofuranne et 470 mg de chlorure de cuivre, on ajoute, peu à peu, une solution 0,9 M de bromure de (4-méthylthio phényl) magnésium. On garde 1 heure à température ambiante. On verse sur une solution glacée de chlorure d'ammonium et extrait avec de l'acétate d'éthyle. Après évaporation sous pression réduite, on chromatographie le résidu sur silice (éluant : cyclohexane-acétate d'éthyle (7-3) puis (1-1)) ; on obtient 5,8 g de produit que l'on dissout dans 40 $cm^3$ de méthanol, 10 $cm^3$ de chlorure de méthylène, 20 $cm^3$ d'acide chlorhydrique 2N. On agite 17 heures à température ambiante puis alcalinise avec du bicarbonate de sodium et extrait avec du chlorure de méthylène. Après évaporation sous pression réduite, on chromatographie le résidu (6,75 g) sur silice (éluant : éther diéthylique-acétate d'éthyle (6-4)). On recristallise le produit brut (3,4 g) dans l'éthanol et obtient 2,93 g de produit recherché $[alpha]_D$ + 125° (c = 1 % $CHCl_3$) (solvaté éthanol).

| Analyse pour $C_{28}H_{32}O_3S$ | | | |
|---|---|---|---|
| | C % | H % | S % |
| Calculé | 74,96 | 7,19 | 7,14 |
| Trouvé | 74,9 | 7,2 | 6,9 |

**EXEMPLE 2 : 17alpha-(3-bromo 1-propynyl) 17béta-hydroxy 11béta-[4-(méthylthio) phényl] estra-4,9-dièn-3-one**

A une solution de 0,9 g de produit de l'exemple 1 (désolvaté par entraînement azéotropique) dans 10 $cm^3$ de chlorure de méthylène, on ajoute 660 mg de tétrabromure de carbone. On refroidit à 0°C et ajoute 707 mg de triphényl phosphine. On agite pendant 25 minutes puis chromatographie le mélange sur silice (éluant : cyclohexane-éther (3-7)), puis éther seul. On obtient 820 mg de produit attendu.

| Analyse pour $C_{28}H_{31}BrO_2S$ | | | | |
|---|---|---|---|---|
| | C % | H % | Br % | S % |
| Calculé | 65,73 | 6,10 | 15,62 | 6,26 |
| Trouvé | 65,1 | 6,1 | 15,1 | 6,0 |

**EXEMPLE 3 : 17alpha-(3-fluoro 1-propynyl) 17béta-hydroxy 11béta-[4-(méthylthio) phényl] estra-4,9-dièn-3-one**

A une solution de 1,6 g de produit obtenu comme à l'exemple 2 dans 32 $cm^3$ d'acétonitrile on ajoute 1,6 g de fluorure de potassium et 1,6 g d'éther couronne (18 crown 6). On chauffe pendant 1 heure 15 au reflux puis évapore à sec sous pression réduite. On reprend le résidu avec du chlorure de méthylène, lave à l'eau, sèche et concentre à sec sous pression réduite. On chromatographie le résidu (1,79 g) sur silice (éluant cyclohexane-éther (1-1)). On obtient 1,05 g de produit attendu.
/alpha/$_D$ = +129,5° (c = 1% $CHCl_3$).

| Analyse pour $C_{28}H_{31}FO_2S$ | | | | |
|---|---|---|---|---|
| | C % | H % | F % | S % |
| Calculé | 74,63 | 6,93 | 4,21 | 7,11 |
| Trouvé | 74,4 | 7,1 | 4,1 | 6,8 |

**EXEMPLE 4 : 17alpha-(3-chloro 1-propynyl) 17béta-hydroxy 11béta-[4-(méthylthio) phényl] estra-4,9-dièn-3-one**

On opère comme à l'exemple 2 à partir de 0,9 g de produit obtenu à l'exemple 1, en utilisant du tétrachlorure de carbone au lieu de tétrabromure de carbone. On obtient 0,77 g de produit recherché.
/alpha/$_D$ = +122° (c = 1% $CHCl_3$).

| Analyse pour $C_{28}H_{31}ClO_2S$ | | | | |
|---|---|---|---|---|
| | C % | H % | S % | Cl % |
| Calculé | 72,00 | 6,68 | 6,86 | 7,59 |
| Trouvé | 72,0 | 7,0 | 6,6 | 7,4 |

Compositions pharmaceutiques.

On a préparé des comprimés répondant à la formule suivante :

| | |
|---|---|
| - Produit de l'exemple 3 | 50 mg |
| - Excipient q.s. pour un comprimé terminé à | 120 mg |
| (Détail de l'excipient : talc, amidon, stéarate de magnésium). | |

Etude pharmacologique des produits de l'invention

Etude de l'activité des produits de l'invention sur les récepteurs hormonaux :

Récepteur progestogène de l'utérus de lapine :

Des lapines impubères d'environ 1 kg reçoivent une application cutanée de 25 microgrammes d'estradiol. 5 jours après ce traitement, les animaux sont sacrifiés, les utérus sont prélevés, pesés et homogénéisés à 0°C, à l'aide d'un Potter teflon-verre dans une solution tamponnée TS (Tris 10mM, saccharose 0,25 M, HCl pH 7,4) (1 g de tissu pour 50 ml de TS). L'homogénat est ensuite ultracentrifugé (105 000 g x 90 mn) à 0°C. Des aliquotes du surnageant ainsi obtenu, sont incubées à 0°C pendant un temps t, avec une concentration constante (T) de Produit R tritié (17,21-diméthyl 19-nor 4,9-pregnadiène-3,20-dione) en présence de concentrations croissantes (0 - 2 500. $10^{-9}$M) soit de R froid, soit de progestérone froide, soit du produit froid à tester. La concentration de R tritié lié (B) est ensuite mesurée dans chaque incubat par la technique d'adsorption au charbon dextran.

Récepteur glucocorticoïde du thymus de rat :

Des rats mâles Sprague Dawley EOPS de 160 à 200 g sont surrénalectomisés. 4 à 8 jours après cette ablation, les animaux sont sacrifiés, et les thymus sont prélevés et homogénéisés à 0°C dans un tampon Tris 10mM, saccharose 0,25M, dithiothreitol 2mM, HCl pH 7,4, à l'aide d'un Potter polytétrafluoroéthylène-verre (1 g de tissu pour 10 ml de TS). L'homogénat est ensuite ultracentrifugé (105 000 g x 90 mn) à 0°C. Des aliquotes du surnageant ainsi obtenu, sont incubées à 0°C pendant un temps (t) avec une concentration constante (T) de dexaméthasone tritiée en présence de concentrations croissantes (O - 2 500.$10^{-9}$M) soit de dexaméthasone froide, soit du produit froid à tester. La concentration de la dexaméthasone tritiée liée (B) est ensuite mesurée dans chaque incubat par la technique d'adsorption au charbon dextran.

Calcul de l'affinité relative de liaison:

Le calcul de l'affinité relative de liaison (ARL) est identique pour tous les récepteurs.

On trace les 2 courbes suivantes : le pourcentage de l'hormone tritiée liée $\frac{B}{T}$ en fonction du logarithme de la concentration de l'hormone de référence froide et $\frac{B}{T}$ en en fonction du logarithme de la concentration du produit froid testé.

On détermine la droite d'équation $I_{50} = (\frac{B}{T}\ max + \frac{B}{T}\ min)/2$. $\frac{B}{T}$ max = Pourcentage de l'hormone tritiée liée pour une incubation de cette hormone tritiée à la concentration (T). $\frac{B}{T}$ min = Pourcentage de l'hormone tritiée liée pour une incubation de cette hormone tritiée à la concentration (T) en présence d'un grand excès d'hormone froide (2500.$10^{-9}$M).

Les intersections de la droite $I_{50}$ et des courbes permettent d'évaluer les concentrations de l'hormone de référence froide (CH) et du produit froid testé (CX) qui inhibent de 50% la liaison de l'hormone tritiée sur le récepteur.

L'affinité relative de liaison (ARL) du produit testé est déterminée par l'équation

$$ARL = 100\ \frac{(CH)}{(CX)}$$

Les résultats obtenus sont les suivants :

| Temps d'incubation à 0 °C | Glucocorticoïde | | Progestogène | |
|---|---|---|---|---|
| Produits des exemples | 1H | 24H | 2H | 24H |
| 1 | 133 | 99 | 72 | 86 |
| 3 | 142 | 169 | 55 | 225 |
| 4 | 156 | 139 | 70 | 351 |

## Revendications

1) Les produits répondant à la formule ($I_{AD}$) :

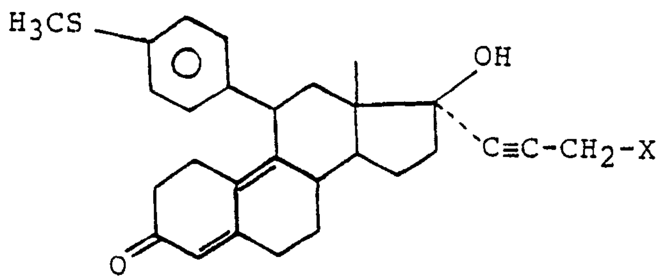

($I_{AD}$)

dans laquelle le substituant X représente un radical hydroxy ou un atome d'halogène.

2) Les produits de formule ($I_{AD}$) telle que définie à la revendication 1 dans laquelle X représente un atome de chlore, de brome ou de fluor.

3) La 17alpha-(3-fluoro 1-propynyl) 17béta-hydroxy 11béta-[4-(méthylthio) phényl] estra-4,9-dien-3-one.

4) A titre de médicaments, les produits définis à l'une quelconque des revendications 1 à 3, pharmaceutiquement acceptables.

5) Les compositions pharmaceutiques renfermant comme principe actif au moins un médicament défini à la revendication 4.

Office européen des brevets

# RAPPORT DE RECHERCHE EUROPEENNE

Numero de la demande

EP 89 40 3142

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | FR-A-2 377 418 (ROUSSEL-UCLAF)<br>* Revendications 1,2,15 *<br>--- | 1,4,5 | C 07 J 31/00<br>A 61 K 31/565 |
| X | STEROIDS, vol. 37, no. 4, avril 1981, pages 361-382, San Francisco, US; A. BELANGER et al.: "Regio and stereospecific synthesis of 11beta-substituted 19-norsteroids"<br>* En entier *<br>--- | 1,4,5 | |
| D,X | WO-A-8 303 099 (ROUSSEL-UCLAF)<br>* Revendications 1,3-5,7,11-13 *<br>----- | 1-5 | |
| | | | **DOMAINES TECHNIQUES RECHERCHES (Int. Cl.5)** |
| | | | C 07 J 31/00 |

Le présent rapport a été établi pour toutes les revendications

| Lieu de la recherche | Date d'achèvement de la recherche | Examinateur |
|---|---|---|
| LA HAYE | 05-02-1990 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)